# EUROPEAN PATENT APPLICATION

(11) **EP 1 400 496 A1**
(43) Date of publication of application: **24.03.2004**
(21) Application number: 03104149.4
(22) Date of filing: 09.10.2000
(51) Int. Cl.: C03C 14/00, C03C 17/00, C07D 209/34, C07D 487/04

(54) **Pigment precursors for making pigmented vitreous material**

(30) Priority: 04.07.2000 EP 00114313
(62) Divisional of application: 00969463.9
(71) Applicant: Ciba Specialty Chemicals Holding Inc., 4057 Basel (CH)
(72) Inventor: Bujard, Patrice, 4153 Reinach (CH); Hall-Goulle, Véronique, 4143 Dornach (CH); Hao, Zhimin, 4125 Riehen (CH); Nagasue, Hitoshi, Hyogo 669-1112 (JP); De Keyzer, Gerardus, 4125 Riehen (CH)

(57) **Abstract**

The present application relates to soluble pigment precursors comprising a partial structure 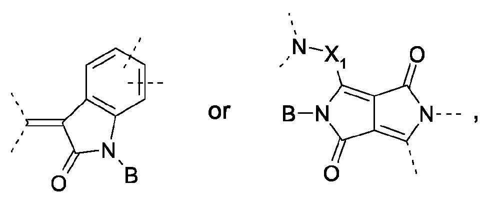 , wherein X₁ is an aromatic or heteroaromatic ring, B is hydrogen or a group of the formula 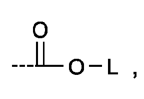 but at least one group B is not hydrogen, and L is a solubilizing group.

## Description

The present application relates to soluble pigment precursors useful for the manufacture of pigmented vitreous materials, preferably in the absence of significant amounts of dispersants. These pigmented vitreous materials can be used as coloured materials for any known purposes. Examples of uses are layers on beverage bottles, TV screens and other glass items.

As pigmented vitreous materials, there are understood materials comprising a crosslinked matrix of polycondensated transition metal oxides or hydroxides (generally referred to in the literature as "sol-gels"), wherein organic pigment particles are entrapped. The matrix may consist essentially of metal-oxygen-metal links, or may also comprise organic links between the metal atoms. The matrix in particular also can be a hybrid organic-inorganic system, for example an ormocer or a ceramer. All such materials are well-known in the art, and are described for example in many patents and patent applications, such as EP 354 465, EP 426 037, EP 504 926 and EP 590 740, as well as also in reviews articles, reference books and technical encyclopedia.

EP 648 770 and EP 648 817 disclose carbamate-functional, soluble chromophors which can be converted to the corresponding pigments by heating them to relatively high temperatures, with the ensuing elimination of the carbamate radicals. These compounds are suitable for the mass colouring of polymers and, according to EP 654 711, for the colouring of resists and of polymer coats to which they are applied. Compounds of the same type but with improved properties are known for example from EP 742 556, WO 98/32802, WO 98/45757, WO 98/58027 and WO 99/01511.

US 5,243,052 discloses carbonates of quinophthalones, which are of limited solubility and can be used in heat-sensitive recording systems. The leuco dye is embedded within a polymer, preferably in polyethyloxazoline.

EP 504 926 discloses a coating solution composition for forming glass gel thin film, color glass gel filter, and display device using the same, wherein colorant material particles are incorporated together with not less than 0.01 weight % of a dispersant, preferably from 5 to 100 parts by weight with respect to 100 parts by weight of the coloring material. The colorant material particles are such of dyes or pigments, for example azo yellow and red, perylene, perinone, dioxazine, thioindigo, isoindolinone, quinophthalone, quinacridone, phthalocyanine or inorganic pigments. The glass gel thin film is formed at the temperature of 100 to 300°C.

Further, similar sol-gel processes and compositions using organic pigment dispersions are disclosed in JP-A-07/207186, JP-A-08/175823, JP-A-09/239311 and JP-A-10/204296.

The dispersant is disclosed to strengthen the gel film layer, so that the negative influence of the colorant particles would be compensated. However, the sol-gels of the prior art do still not match satisfactory today's high requirements in workability, strength, homogeneity, light, heat and moisture stability, transparency and coloristics. The colorant is not entirely sealed within the inorganic gel, so that it is exposed to oxygen and moisture and partially extracted by chemicals used in the manufacture of articles comprising the gels. High concentrations of colorant furthermore require high amounts of dispersants, leading to further impaired properties. A key limitation is that organic pigments of different classes cannot satisfactory be used together because they require different, often antagonistic dispersants.

The instant invention surprisingly leads to remarkably improved properties through the use of certain soluble organic pigment precursors which thermally split to insoluble organic pigments. Highly unexpected, the pigment particles are strongly bonded to the gel especially in the absence of an additional dispersant. Surfactants may nevertheless be added, for example to improve the surface quality, but advantageously they are only optional and do not need to be adapted to the pigment. Althought the real mechanism is not elucidated yet, it is believed that the pigment's solubilizing groups do interfere with the gel formation mechanism so that the gel's affinity to organic pigments is improved, instead of just splitting off into an olefin and carbon dioxide as is generally the case in solution.

The invention pertains to a 2,5-dihydro-pyrrolo[3,4-c]pyrrole-1,4-dione of the formula 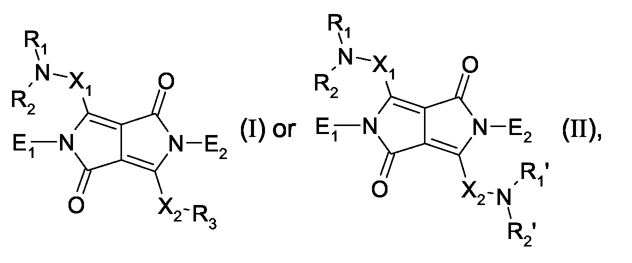 in which X₁ and X₂ independently of one another are a divalent aromatic radical of the formula 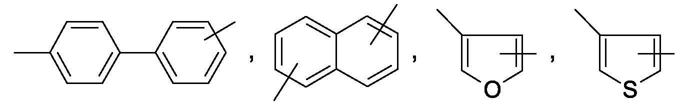 or 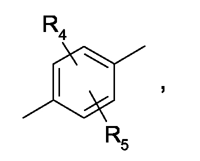 R₃ is a radical CN, COR₆, CO₂R₆, CON(R₆)₂, NO₂, SO₂R₆, SOR₆, SO₂N(R₆)₂ or PO(OR₇)₂ ,
R₄ and R₅ independently of one another are hydrogen, chlorine, bromine, methyl, ethyl, methoxy or ethoxy,
R₇ is C₁-C₆alkyl or phenyl,
R₁, R₂, R₁', R₂' and R₆ independently of one another are hydrogen, C₁-C₁₈alkyl or C₁-C₁₈alkenyl which is unsubstituted or substituted by hydroxy, mercapto, C₁-C₆alkoxy or C₁-C₆alkylmercapto, or phenyl which is unsubstituted or substituted by chlorine, bromine, hydroxy, C₁-C₆alkyl, C₁-C₆alkoxy, C₁-C₆alkylmercapto, CN, NO₂ or CF₃,
or R₁ and R₂ or R₁' and R₂', together with the nitrogen atom to which they are attached, form a 5- or 6-membered heterocyclic radical which is unsubstituted or substituted by C₁-C₆alkyl or phenyl and is selected from the group consisting of pyrrolidinyl, piperidyl, pyrrolyl, triazolyl, imidazolyl, pyrazolyl, piperazinyl, morpholinyl, thiomorpholinyl, carbazol-1-yl, indol-1-yl, indazol-1-yl, benzimidazol-1-yl, tetrahydroquinol-1-yl and tetrahydroquinol-2-yl, or,
if R₁ or R₁' is hydrogen, R₂ or R₂' is a radical of the formula 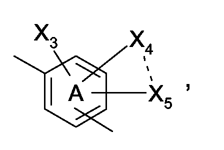 in which X₃ and X₄ independently of one another are hydrogen, chlorine, bromine, NO₂, methyl, methoxy or ethoxy and X₅ and X₆ form a 5- or 6-membered heterocyclic ring which together with A produces a benzimidazolonyl, dihydroxyquinazolinyl, quinolonyl, benzoxazolonyl, phenmorpholonyl, quinazolinonyl or phthalimidyl radical or a radical of the formula 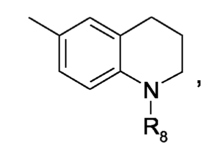 in which R₈ is C₁-C₆alkyl or phenyl, or X₂-R₃ can be a radical 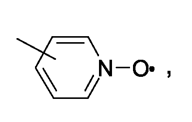 E₁ is hydrogen and E₂ is a group B, E₁ is a group B and E₂ is hydrogen, or E₁ and E₂ are both a group B,
B is a group of the formula 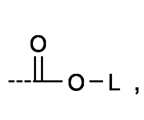 and
L is any suitable solubilizing group,
with the proviso that when said 2,5-dihydro-pyrrolo[3,4-c]pyrrole-1,4-dione is of formula (II), R₁ is not C₁-C₁₈alkylamino.

Compounds which include at least one 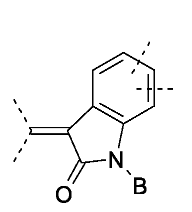 moiety in their structure are new and are also an object of the invention.

The instant compounds can be used in a process for making a pigmented vitreous material from a liquid or dissolved transition metal compound, wherein the liquid or dissolved transition metal compound reacts to form crosslinks between the liquid or dissolved transition metal atoms in the pigmented vitreous material, also comprising a dissolved compound of the formula (I) or (II), or including at least one 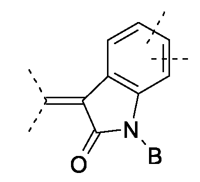 moiety in its structure, and the vitreous material is heated so that said dissolved compound is transformed into a pigment.

Transition metals are well-known in the art and may for example be aluminium, zinc, zirconium, titanium, iron, cobalt and nickel, and very particularly silicium. The liquid or dissolved transition metal compounds are also well-known in the art and may for example be an alkoxide or a mixed oxide/alkoxide, which may in addition contain further substituents, for example C₁-C₄alkyl groups or halogens.

The transformation of the compound of the formula (I) or (II) into a pigment by heating may be performed simultaneously with the liquid or dissolved transition metal compound's crosslinking reaction, or as a separate final step.

Besides the product's excellent properties, the process of the invention has also the advantage that it is much faster than the prior art, due to the fact that a dispersion step is not necessary. Moreover, the reaction can be conducted at higher temperature and at higher pigment contents in the substantial absence of dispersants, without impairing the transparency, hue and chroma.

The reaction is generally performed in the way, that all ingredients are first mixed to form a composition which can be applied as desired and heated to regenerate the pigment. The composition may also contain effective amounts of a catalyst, for example an acid or a precursor which forms an acid upon heating. The acid or precursor may be added at the time of the composition's preparation, or preferably just before the composition's application.

An effective amount of a catalyst is any quantity suitable to start or accelerate the reaction. Catalysts and the suitable quantities thereof are well-known in the art. Examples are mineral acids, such as hydrochloric acid or nitric acid, Lewis acids, such as boron trifluoride, organic acids, such as formic, acetic or oxalic acid, or the like, preferably with a pKₐ of 3 or lower.

In addition or even instead of catalysts, it is also well-known to use a light source or to heat the mixture to a mild temperature, for example about 50 to 80°C, in order for the crosslinking reaction (gelation) to start.

Before or during gelation, it is possible to work the composition into the desired form by usual means, for example coating layers by spin coating or by printing methods, such as for example screen or inkjet printing. In analogy to the resist technology (disclosed for example in EP 654 711), it is also possible to perform the crosslinking only in specified areas by using for example a laser or arrays of thermoelements, so that an image is obtained which can be developed for example by washing out the ungelated areas with a suitable solvent.

After the gelation is completed, the material is if necessary further converted to a vitreous form by heating to a higher temperature, at which the pigment will also be regenerated.

The instant pigmented vitreous materials have a high transparency, a high resistance to water, solvent and chemicals as well as also a good resistance to shearing and scratching, and particularly a very high thermal stability even under very severe conditions. Due to their high pigment content, they can be applied in very thin, highly transparent and low scattering layers, the thickness of which is from 0.1 to 3 µm.

The crosslinks between the transition metal atoms may consist for example of oxygen, alkylene, oxyalkylene or oxyalkyleneoxy bridges, which may be unsubstituted or further substituted, for example by oxa or fluoro. Polymeric materials may also be used which have suitable substituents, such as -OH groups, may also be used for crosslinking.

Suitable solvents are water or, preferably, any desired protic or aprotic solvents, examples being hydrocarbons, alcohols, amides, nitriles, nitro compounds, sulphur derivatives, N-heterocycles, ethers, ketones and esters which may also be either mono- or polyunsaturated or chlorinated: examples are methanol, ethanol, isopropanol, n-butanol, isobutanol, 2-butanol, diethyl ether, acetone, methyl ethyl ketone, methyl isobutyl ketone, 1,2-dimethoxyethane, 1,2-diethoxyethane, 2-n-butoxyethanol, 2-methoxyethanol, 2-ethoxyethanol, ethyl acetate, tetrahydrofuran, dioxane, acetonitrile, benzonitrile, nitrobenzene, N,N-dimethylformamide, N,N-dimethylacetamide, dimethyl sulfoxide, sulfolane, N-methylpyrrolidone, pyridine, picoline, quinoline, dichloroethane, trichloroethane, benzene, toluene, xylene, anisole and chlorobenzene. Further examples of solvents are described in numerous tabular and reference works. Instead of a single solvent it is also possible to employ mixtures of two or more solvents.

The transition metal compounds which undergo crosslinking act themselves as solvents, too. The ratio of transition metal compound to other solvents is preferably from 25:1 to 2.5:1.

The concentration of the pigment precursor in water or a solvent is usually from 0.01 % by weight, based on the weight of the solution, to approximately 99% by weight of the saturation concentration, it also being possible in certain cases to employ supersaturated solutions without premature precipitation of the solvate. For many pigment precursors the optimum concentration is around ∼0.05-30% by weight, often around 0.1-15% by weight, based on the weight of the solution.

Conversion of the pigment precursor into the pigmentary form takes place by thermal fragmentation, which can be carried out simultaneously with the gel formation or as a subsequent treatment at higher temperature. Thus, the pigment precursor's fragmentation temperature is preferably in the same range as the gel formation, adequately from 50 to 400°C, preferably from 100 to 300°C, most preferably from about 150 to about 250°C.

By an effectively colouring amount is meant that amount which is sufficient to bring about a colour difference ΔE* (CIE-L*a*b*) of ≥ 2 when the pigmented vitreous material is compared with the similarly made, unpigmented vitreous material under the standard illuminant D₆₅ and at a viewing angle of 10°. This amount is preferably from 0.01 to about 50% by weight, more preferably from 0.1 to 30% by weight, most preferably from 10 to 25% by weight, based on the weight of the pigmented vitreous material. It is generally preferred to have a pigment concentration as high as possible, without the vitreous material's properties to be impaired.

The pigment precursors can be employed individually or preferably in mixtures with other pigment precursors. Where the pigment precursors are employed in mixtures, the components of the mixture are preferably those whose colour in the pigmentary form is red, blue, yellow, green, orange or black, more preferably bluish red, orange or black. Preferably, the pigmented vitreous material contains each effective pigmenting amounts of from 2 to 10 organic pigments, preferably from 2 to 5 organic pigments.

Examples of other pigment precursors are known for example from EP 742 556, WO 98/32802, WO 98/45757, WO 98/58027, WO 99/01511, WO 00/17275 and subject-matter of the application PCT/EP-00/03085, as well as from many other publications cited therein, the contents of all are expressly incorporated herein by reference. They preferably contain at least one directly adjacent or conj ugated carbonyl group at each heteroatom attached to groups B.

Bluish red is a wide colour range comprising also colours for which sometimes other designations are used, such as for example magenta, ruby, claret, cabernet, maroon and violet. An especially preferred bluish red has an absorption maximum (λₘₐₓ) from 550 to 590 nm, particularly preferred from 560 to 570 nm or from 570 to 580 nm.

Black is preferably a mixture of each a blue and yellow component and from one to 3 red components, especially any mixture of each any C.I. Pigment Blue and Pigment Yellow and any one C.I. Pigment Red or any combination of two or three C.I. Pigment Red and/or Pigment Violet, examples of which are given below. The optimal ratio of the components depends on the respective spectra and should be chosen so that the absorption is nearly the same at all wavelength from 400 to 700 nm, whereby eventual transmission in some areas may be compensated by additional pigments, the absorption maxima of which correspond to the transmission to be reduced.

Particularly preferred are 3-methylidene-2,3-dihydro-indol-2-on derivatives (such as those disclosed in WO-00/24736 wherein R₁ is H) and 2,5-dihydro-pyrrolo-[3,4-c]pyrrole-1,4-dione derivatives having in the 3 and/or 6 position a rest substituted by at least one amino group (such as those disclosed in EP-B-353184 or EP-A-755933). The contents of WO-00/24736, EP-B-353184 and EP-A-755933 are incorporated herein by reference. These pigments, pure or in combination with other pigments, are particularly suitable for obtaining violet or green colourations. They also lead to surprisingly improved performance in vitreous materials.

Particularly preferred chromophore is a 3-methylidene-2,3-dihydro-indol-2-on derivative of the formula

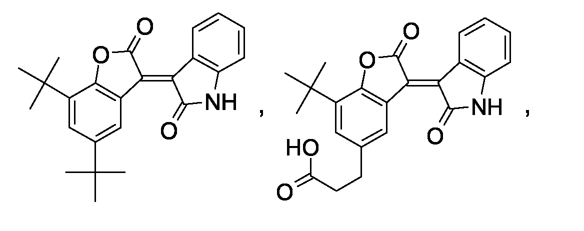

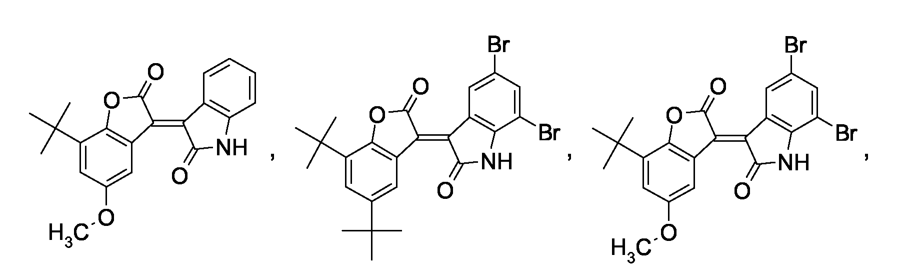

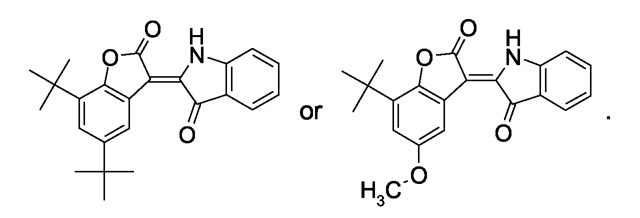

Other 3-methylidene-2,3-dihydro-indol-2-on derivatives having other substituents are described for example in the above-cited reference WO-00/24736.

Preferably -L is a group of the formula 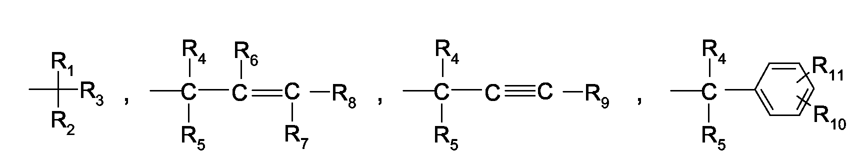 or 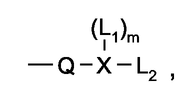 in which R₁, R₃ and R₂ independently of one another are C₁-C₆alkyl,
R₄ and R₅ independently of one another are C₁-C₆alkyl, O, S or N(R₁₂)₂-interrupted C₁-C₆alkyl, unsubstituted or C₁-C₆alkyl-, C₁-C₆alkoxy-, halo-, cyano- or nitro-substituted phenyl or biphenylyl,
R₆, R₇ and R₈ independently of one another are hydrogen or C₁-C₆alkyl,
R₉ is hydrogen, C₁-C₆alkyl or a group of the formula 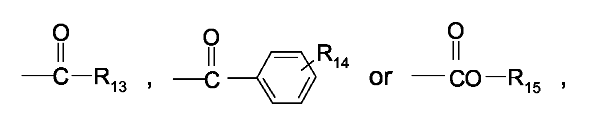 R₁₁ and R₁₀ independently of one another are hydrogen, C₁-C₆alkyl, C₁-C₆alkoxy, halogen, cyano, nitro, N(R₁₂)₂, unsubstituted or halo-, cyano-, nitro-, C₁-C₆alkyl- or C₁-C₆alkoxy-substituted phenyl,
R₁₂ and R₁₃ are C₁-C₆alkyl, R₁₄ is hydrogen or C₁-C₆alkyl and R₁₅ is hydrogen, C₁-C₆alkyl, unsubstituted or C₁-C₆alkyl-substituted phenyl,
Q is p,q-C₂-C₆alkylene which is unsubstituted or substituted one or more times by C₁-C₆alkoxy, C₁-C₆alkylthio or C₂-C₁₂dialkylamino, p and q being different numeric locants,
X is a heteroatom selected from the group consisting of N, O and S, where m is 0 if X is O or S and is 1 if X is N, and
L₁ and L₂ independently of one another are unsubstituted or mono- or poly-C₁-C₁₂alkoxy-, -C₁-C₁₂alkylthio-, -C₂-C₂₄dialkylamino-, -C₆-C₁₂aryloxy-, -C₆-C₁₂arylthio-, -C₇-C₂₄alkylarylamino- or -C₁₂-C₂₄diarylamino-substituted C₁-C₆alkyl or
[-(p',q'-C₂-C₆alkylene)-Z-]ₙ-C₁-C₆alkyl, where n is a number from 1 to 1000, p' and q' are different numeric locants, each Z independently of the others is a heteroatom O, S or C₁-C₁₂alkyl-substituted N, and C₂-C₆alkylene in the repeating units [-C₂-C₆alkylene-Z-] can be identical or different,
and L₁ and L₂ can be saturated or mono- to deca-unsaturated, uninterrupted or interrupted in any desired points by from 1 to 10 groups selected from the group consisting of -(C=O)- and -C₆H₄-, and may carry no or 1 to 10 further substituents selected from the group consisting of halogen, cyano and nitro.

Of particular interest are compounds of the formula (I) or (II) in which L is C₁-C₆alkyl or particularly 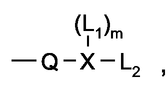 in which Q is C₂-C₄alkylene and L₁ and L₂ are [-C₂-C₁₂alkylene-Z-]ₙ-C₁-C₁₂alkyl or are C₁-C₁₂alkyl which is substituted one or more times by C₁-C₁₂alkoxy, C₁-C₁₂alkylthio or C₂-C₂₄dialkylamino, and m and n are as defined above.

Of very particular interest are compounds of the formula (I) or (II) in which L is C₄-C₅alkyl (especially tert.-butyl or tert.-amyl) or particularly 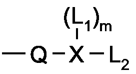 , in which Q is C₂-C₄alkylene, X is O and m is zero, and L₂ is [-C₂-C₁₂alkylene-O-]ₙ-C₁-C₁₂alkyl or is C₁-C₁₂alkyl which is substituted one or more times by C₁-C₁₂alkoxy, especially those in which -Q-X- is a group of the formula -C(CH₃)₂-CH₂-O-.

Alkyl or alkylene can be straight-chain, branched, monocyclic or polycyclic.

Thus C₁-C₁₂alkyl is, for example, methyl, ethyl, n-propyl, isopropyl, n-butyl, sec-butyl, isobutyl, tert-butyl, cyclobutyl, n-pentyl, 2-pentyl, 3-pentyl, 2,2-dimethylpropyl, cyclopentyl, cyclohexyl, n-hexyl, n-octyl, 1,1,3,3-tetramethylbutyl, 2-ethylhexyl, nonyl, trimethylcyclohexyl, decyl, menthyl, thujyl, bornyl, 1-adamantyl, 2-adamantyl or dodecyl.

If C₂-C₁₂alkyl is mono- or polyunsaturated it is C₂-C₁₂alkenyl, C₂-C₁₂alkynyl, C₂-C₁₂alkapolyenyl or C₂-C₁₂alkapolyynyl in which two or more double bonds may if appropriate be isolated or conjugated, examples being vinyl, allyl, 2-propen-2-yl, 2-buten-1-yl, 3-buten-1-yl, 1,3-butadien-2-yl, 2-cyclobuten-1-yl, 2-penten-1-yl, 3-penten-2-yl, 2-methyl-1-buten-3-yl, 2-methyl-3-buten-2-yl, 3-methyl-2-buten-1-yl, 1,4-pentadien-3-yl, 2-cyclopenten-1-yl, 2-cyclohexen-1-yl, 3-cyclohexen-1-yl, 2,4-cyclohexadien-1-yl, 1-*p*-menthen-8-yl, 4(10)-thujen-10-yl, 2-norbornen-1-yl, 2,5-norbornadien-1-yl, 7,7-dimethyl-2,4-norcaradien-3-yl or the various isomers of hexenyl, octenyl, nonenyl, decenyl or dodecenyl.

C₂-C₄alkylene is, for example, 1,2-ethylene, 1,2-propylene, 1,3-propylene, 1,2-butylene, 1,3-butylene, 2,3-butylene, 1,4-butylene or 2-methyl-1,2-propylene. C₅-C₁₂alkylene is, for example, an isomer of pentylene, hexylene, octylene, decylene or dodecylene.

C₁-C₁₂alkoxy is O-C₁-C₁₂alkyl, preferably O-C₁-C₄alkyl.

C₆-C₁₂aryloxy is O-C₆-C₁₂aryl, for example phenoxy or naphthoxy, preferably phenoxy.

C₁-C₁₂alkylthio is S-C₁-C₁₂alkyl, preferably S-C₁-C₄alkyl.

C₆-C₁₂arylthio is S-C₆-C₁₂aryl, for example phenylthio or naphthylthio, preferably phenylthio.

C₂-C₂₄dialkylamino is N(alkyl₁)(alkyl₂), where the sum of the carbon atoms in the two groups alkyl₁ and alkyl₂ is from 2 to 24, preferably N(C₁-C₄alkyl)-C₁-C₄alkyl.

C₇-C₂₄alkylarylamino is N(alkyl₁)(aryl₂), where the sum of the carbon atoms in the two groups alkyl₁ and aryl₂ is from 7 to 24, for example methylphenylamino, ethylnaphthylamino or butylphenanthrylamino, preferably methylphenylamino or ethylphenylamino.

C₁₂-C₂₄diarylamino is N(aryl₁,)(aryl₂), where the sum of the carbon atoms in the two groups aryl₁ and aryl₂ is from 12 to 24, for example diphenylamino or phenylnaphthylamino, preferably diphenylamino.

C₆-C₁₆aryl ist for example phenyl, naphthyl, anthracenyl, pyrenyl or naphthacenyl.

C₇-C₂₄aralkyl ist any group comprising at least each an alkyl and an aryl part, for example benzyl, phenethyl, tolyl, dodecylphenyl, indanyl or acenaphtenyl.

Halogen is chlorine, bromine, fluorine or iodine, preferably fluorine or chlorine, most preferably fluorine. n is preferably a number from 1 to 100, with particular preference a number from 2 to 12.

Particularly preferred pigment precursors are such wherein all B are identical groups of the formula 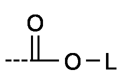 and for each of above chromophores L is tert.-butyl, tert.-amyl, 3-methoxy-2-methyl-2-propyl, 3-(2'-methoxy-ethoxy)-2-methyl-2-propyl, 2-methyl-3-butin-2-yl or 3-methyl-2-buten-1-yl.

In addition to the pigment, the vitreous material may also contain other compounds such as additives known in the art to improve the material's properties such as UV absorbers or transparency improvers.

Heating can be carried out by any desired thermal means, including irradiation; for example, by treatment in a thermal oven or by electromagnetic radiation, for example IR or NIR radiation, or laser pulses or microwaves.

The heating time for regenerating the pigment is not critical, as long as care is taken that it is sufficiently long for the fragmentation of the pigment precursor to be completed. Typically, it ranges from several seconds to several hours, preferably from about 1 to about 30 minutes.

The suitable heating time for gel formation depends on the temperature according to the laws of thermodynamics, so that it can simply be determined depending on the desired temperature.

The heating temperature, however, should be evaluated carefully. In general, it is appropriate to use a temperature from 150 to 300°C, especially from about 180°C to 250°C. Preferred temperatures are from 180°C to 200°C. Together with the solubilizing group and the solvent used for gel formation, the temperature influences the crystal structure of the pigment which is formed. Typically, one should run parallel experiments at 150°C and 200°C to check for eventual coloristic differences.

Surprisingly, it has been found that the use of a pigment precursor in which L is 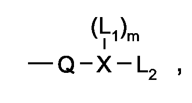 together with a solvent for gel formation of structure 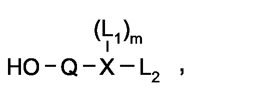 leads to improvements in colour after heating to a temperature from 150 to 300°C to form the vitreous material. The compound may be added at once or also in portions, at any time from the beginning to just before the heating step, in any amounts. Preferably, the weight ratio of such compound to compound (I) or (II) is from 1:4 to 100:1.

Preferred solvents for gel formation are 2-C₁-C₁₂alkoxyethanol, 2-C₁-C₁₂alkoxypropanol, 2,3-di-C₁-C₁₂alkoxypropanol, diethylenglycol-mono-C₁-C₁₂alkyl ether, triethylenglycol-mono-C₁-C₁₂alkyl ether, dipropylenglycol-mono-C₁-C₁₂alkyl ether, tripropylenglycol-mono-C₁-C₁₂alkyl ether, or diethylenglycol, triethylenglycol, dipropylenglycol or tripropylenglycol each monoesterified with a C₁-C₁₂ carboxylic acid.

When a catalyst is used in the form of its precursor, the catalyst precursor can for example be of formula 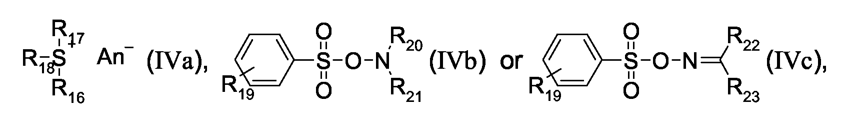 wherein R₁₆, R₁₇ and R₁₈ are independently from one another C₁-C₂₄alkyl, C₆-C₂₄aryl or C₇-C₂₄aralkyl,
R₁₉ is C₁-C₂₄alkyl,
R₂₀, R₂₁, R₂₂ and R₂₃ are independently from one another C₁-C₂₄alkyl, C₆-C₂₄aryl or C₇-C₂₄aralkyl,
or R₂₀ and R₂₁ together or R₂₂ and R₂₃ together are C₄-C₂₄alkylen, C₄-C₂₄aralkylen, 3-oxa-pentylen or N-C₁-C₂₄alkyl-3-aza-pentylen,

An is PX₆, AsX₆, SbX₆, BX₄, R₂₄-SO₃, R₂₄-OSO₃ or R₂₅-PO₃R₂₆, wherein R₂₄, R₂₅ and R₂₆ are independently from one another C₁-C₂₄alkyl, C₆-C₂₄aryl or C₇-C₂₄aralkyl . R₂₅ and R₂₆ are preferably C₁-C₂₄alkyl, most preferably C₁-C₄alkyl, particularly methyl, and X is halogen.

Further substituents may be present in the compounds of formulae (IVa), (IVb) and (IVc), for example halogen atoms or nitro or C₁-C₂₄alkoxy groups.

Of course, the instant new 3-methylidene-2,3-dihydro-indol-2-on and amino-substituted 2,5-dihydro-pyrrolo[3,4-c]pyrrole-1,4-dione colorants may also be used in other fields, for example as fluorescent colorants or as latent pigments, such as described for example in US-5,484,943, EP-B-0 648 817, US-5,879,855, WO98/45756, WO98/45757, WO98/58027, WO00/27930 and EP-A-1 044 945.

The examples which follow illustrate the invention, without limiting its scope in any way.

### Example 1:

5.3 g of di-tert-butyldicarbonate are added to a suspension of 5 g of 3,6-bis-(4-diphenylamino-phenyl)-2,5-dihydro-pyrrolo[3,4-c]pyrrole-1,4-dione and 0.3 g of dimethylaminopyridine in 150 ml of tetrahydrofuran. After stirring overnight at room temperature, the reaction mixture was filtered through a small amount of kieselguhr. Addition of hexane to the residue followed by filtration provides 5.3 g of a compound having the formula:

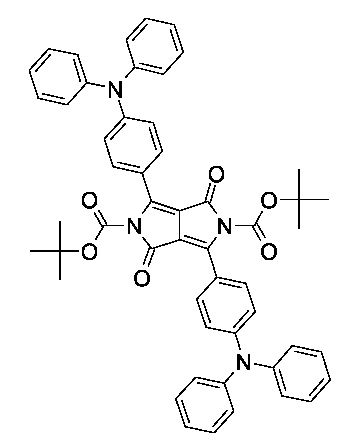

Analysis: calc.: C 75.89%, H 5.63%, N 6.81%, O 11.66%; found: C 74.96%, H 5.79%, N 6.64%, O 11.88%. Midpoint decomposition temperature: 184.7°C; weight loss (calc.) = 24.3%, weight loss (found) = 26.06%.

### Example 2:

8.8 g of di-tert-amyldicarbonate are added to a suspension of 100 g of 3,7-bis-(2-oxo-1,2-dihydro-indol-3-ylidene)-3,7-dihydro-benzo[1,2-.b.;4,5-.b.']difuran-2,6-dione and 8.3 g of dimethylaminopyridine in 1250 ml of dimethylacetamide. After stirring overnight at room temperature, the reaction mixture is filtered through a small amount of kieselguhr and the filtrate is evaporated. Tetrahydrofuran and hexane are added to the residue, and filtration followed by drying provides 57.8 g (39% of theory) of a dark compound having the formula 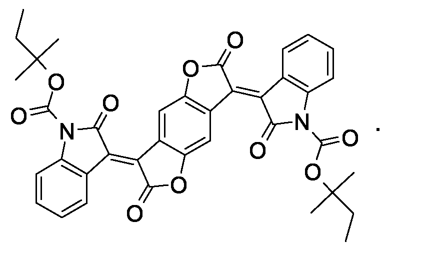 Midpoint decomposition temperature: 133.1°C; weight loss (calc.) = 33.7%, weight loss (found) = 28.6%.

### Example 3:

A mixture of 2.0 g tetraethoxysilane and 0.5 g of phenyltriethoxysilane is dissolved into a mixture of 12.5 g of ethanol and 3.75 g of 1 M-HCl. After 2 to 4 hours of hydrolysis, the reaction mixture is added dropwise to a solution of 0.40 g of the product of example 1 in 3.75 g of cyclohexanone. After dilution with 20 g of isopropanol, the resulting solution is microfiltrated through a 0.45 µm filter, then spin-coated onto a glass substrate (first 10 s at 100 rpm, then 30 s at 500 rpm). The coated glass plate is dried for 2 min at 100°C, then heated further for 5 min at 200°C, whereby the colour changes from orange-yellow to violet.

### Example 4:

7 parts by weight of tetraethoxysilane, 1.3 parts by weight of nitric acid in 1.5 part by weight of water, 0.5 parts by weight of the product of example 2 in 89.89 parts by weight of water are mixed together to form a coating solution which is coated onto a cleaned glass plate by spin coating. After drying for 20 minutes at 80°C, the plate is heated to 200°C for 20 minutes. A uniform coating is obtained on the glass plate, the absorbtion maximum of which is at about 760-765 nm.

### Example 5:

Following components are mixed:
■ 25 parts by weight of the product of example 2;
■ 25 parts by weight of the product of formula 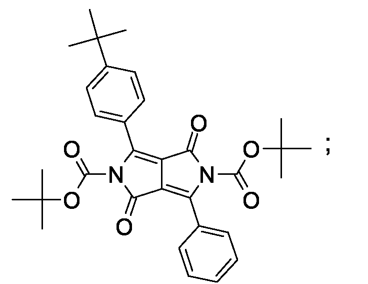
■ 25 parts by weight of the product of formula 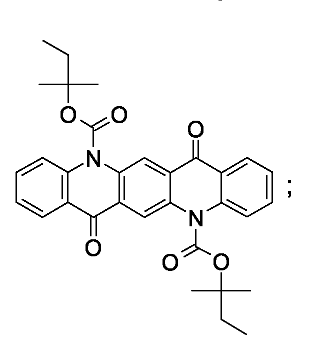
■ 25 parts by weight of the product of formula

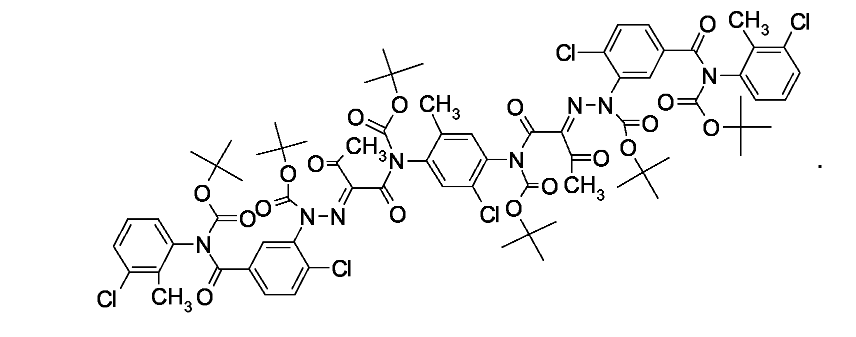

### Example 6:

It is proceeded as in example 4, with the difference that dioxane is used as a solvent instead of water and the mixture of example 5 is used instead of the product of example 2. A uniform black coating is obtained.

## Claims

1. A 2,5-dihydro-pyrrolo[3,4-c]pyrrole-1,4-dione of the formula 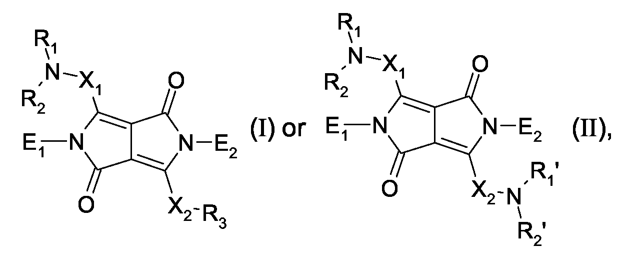 in which X₁ and X₂ independently of one another are a divalent aromatic radical of the formula 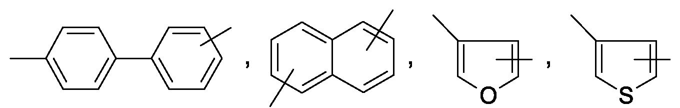 or 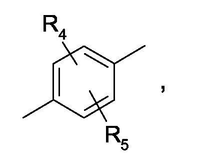 R₃ is a radical CN, COR₆, CO₂R₆, CON(R₆)₂, NO₂, SO₂R₆, SOR₆, SO₂N(R₆)₂ or PO(OR₇)₂ ,
R₄ and R₅ independently of one another are hydrogen, chlorine, bromine, methyl, ethyl, methoxy or ethoxy,
R₇ is C₁-C₆alkyl or phenyl,
R₁, R₂, R₁', R₂' and R₆ independently of one another are hydrogen, C₁-C₁₈alkyl or C₁-C₁₈alkenyl which is unsubstituted or substituted by hydroxy, mercapto, C₁-C₆alkoxy or C₁-C₆alkylmercapto, or phenyl which is unsubstituted or substituted by chlorine, bromine, hydroxy, C₁-C₆alkyl, C₁-C₆alkoxy, C₁-C₆alkylmercapto, CN, NO₂ or CF₃,
or R₁ and R₂ or R₁' and R₂', together with the nitrogen atom to which they are attached, form a 5- or 6-membered heterocyclic radical which is unsubstituted or substituted by C₁-C₆alkyl or phenyl and is selected from the group consisting of pyrrolidinyl, piperidyl, pyrrolyl, triazolyl, imidazolyl, pyrazolyl, piperazinyl, morpholinyl, thiomorpholinyl, carbazol-1-yl, indol-1-yl, indazol-1-yl, benzimidazol-1-yl, tetrahydroquinol-1-yl and tetrahydroquinol-2-yl, or,
if R₁ or R₁' is hydrogen, R₂ or R₂' is a radical of the formula 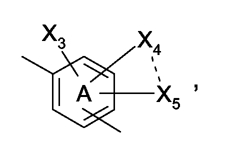 in which X₃ and X₄ independently of one another are hydrogen, chlorine, bromine, NO₂, methyl, methoxy or ethoxy and X₅ and X₆ form a 5- or 6-membered heterocyclic ring which together with A produces a benzimidazolonyl, dihydroxyquinazolinyl, quinolonyl, benzoxazolonyl, phenmorpholonyl, quinazolinonyl or phthalimidyl radical or a radical of the formula 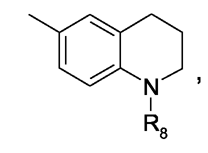 in which R₈ is C₁-C₆alkyl or phenyl,
or X₂-R₃ can be a radical 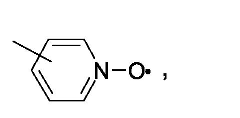 E₁ is hydrogen and E₂ is a group B, E₁ is a group B and E₂ is hydrogen, or E₁ and E₂ are both a group B,
B is a group of the formula 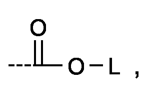 and
L is any suitable solubilizing group,
with the proviso that when said 2,5-dihydro-pyrrolo[3,4-c]pyrrole-1,4-dione is of formula (II), R₁ is not C₁-C₁₈alkylamino.

2. A compound having at least one 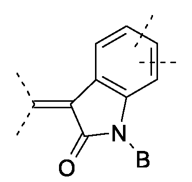 moiety in its structure, wherein B is a group as defined in claim 1.
